(19) Europäisches Patentamt
European Patent Office
Office européen des brevets

(11) **EP 3 349 706 B1**

(12) **EUROPEAN PATENT SPECIFICATION**

(45) Date of publication and mention
of the grant of the patent:
**03.11.2021 Bulletin 2021/44**

(21) Application number: **16770629.0**

(22) Date of filing: **15.09.2016**

(51) Int Cl.:
**A61F 13/49** *(2006.01)*

(86) International application number:
**PCT/US2016/051802**

(87) International publication number:
**WO 2017/048883 (23.03.2017 Gazette 2017/12)**

(54) **TAPED ABSORBENT ARTICLE HAVING A BELT PORTION**

VERKLEBTER SAUGFÄHIGER ARTIKEL MIT EINEM GURTTEIL

ARTICLE ABSORBANT COLLÉ AYANT UNE PARTIE CEINTURE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.09.2015 US 201562220265 P**

(43) Date of publication of application:
**25.07.2018 Bulletin 2018/30**

(73) Proprietor: **The Procter & Gamble Company
Cincinnati, OH 45202 (US)**

(72) Inventors:
• **GREENING, Nelson, Edward, II
Cincinnati, Ohio 45202 (US)**
• **SHEEHAN, Astrid, Annette
Cincinnati, Ohio 45202 (US)**

(74) Representative: **P&G Patent Germany
Procter & Gamble Service GmbH
Sulzbacher Straße 40
65824 Schwalbach am Taunus (DE)**

(56) References cited:
**WO-A1-2014/185242      US-A1- 2013 211 357
US-A1- 2013 255 861      US-A1- 2013 306 226
US-A1- 2013 310 785**

**Description**

FIELD

The present disclosure is generally related to absorbent articles having a belt portion, and, is more specifically related to, taped absorbent articles having a belt portion.

BACKGROUND

Taped absorbent articles for personal hygiene, such as disposable diapers for infants and toddlers or adult incontinence undergarments are designed to absorb and contain quantities of urine and BM (hereafter together "bodily exudates"). These taped absorbent articles may comprise a chassis comprising several layers providing different functions, for example, a topsheet, a backsheet, and an absorbent core disposed between the topsheet and the backsheet, among other layers, if desired. The taped absorbent articles may also comprise ears comprising fasteners and landing zone regions configured to receive the fasteners for product application.

Some absorbent articles may be in the form of pants. The pants may either have permanent or refastenable side seams. Pants are typically applied and removed like underwear by pulling the pant up a wearer's legs and over the buttocks and hips to the wearer's waist. As such, some pants require high pre-strains in waist elastics to be able to expand to fit over the hips and buttocks and then contract when positioned around the wearer's waist to provide a snug fit. In some instances, a belt attached to a chassis may be used to provide such pre-strains. The chassis may comprise at least a topsheet, a backsheet, and an absorbent core. The chassis may have a lateral axis defining a front region and a rear region of an absorbent article. A first belt portion may be joined to the chassis in the front region and a second belt portion may be joined to the chassis in the rear region. Together the first and second belt portions form the belt. The belts typically comprise a plurality of laterally extending elastic strands across a width of the belt. The elastic strands may be sandwiched between two layers of nonwoven or other materials. The elastic profiles of the belt of the pants are not designed for or suitable for taped absorbent article-style application.

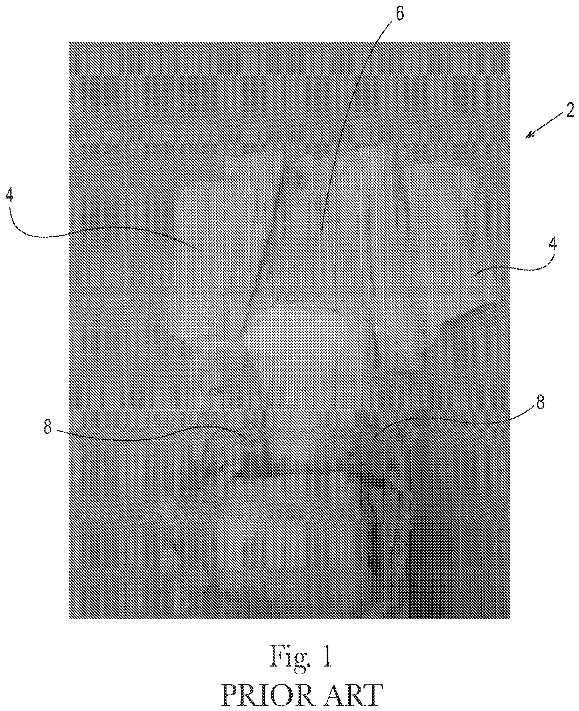

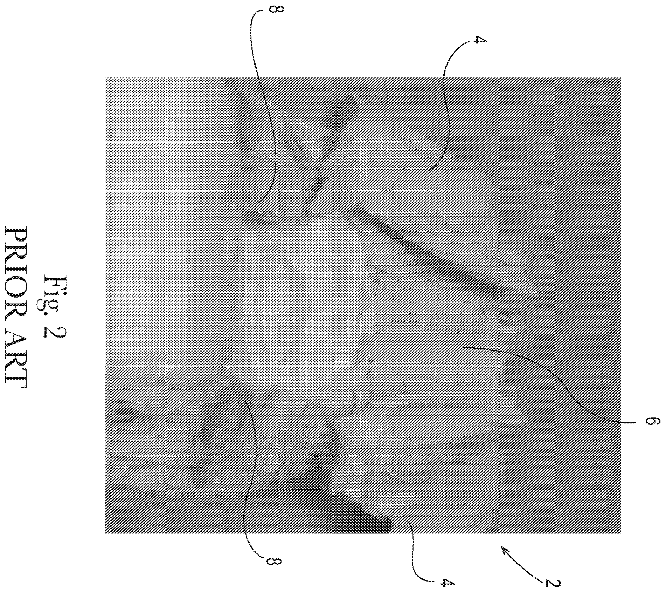

Taped absorbent articles, on the other hand, are not pulled up the legs, over the hips and buttocks, to the waist, but, instead are typically applied when a wearer is lying on his or her back. The taped absorbent articles are first placed on a changing table or other flat surface and then the wearer (typically a baby or small child) is positioned over the absorbent article. Typical taped absorbent articles have discrete back ears that are joined to a chassis. These back ears have a tendency to not lie flat owing to the elastic contractions in the waist band and leg cuffs and the waist band's overlap with the chassis. This can cause difficulties in a caregiver applying the taped product, as the caregiver needs to try to fight the elastic contraction and force the ears into a flat configuration for application of the absorbent article. Referring to Figs. 1 and 2, one example of a taped absorbent article 2 having these issues is Huggies® Little Movers by Kimberly-Clark. The ears 4 are discrete and do not lie flat owing to waist band 6 and leg elastic 8 contractions and the fact that the waist band overlaps the chassis. Further, the ears 4 may comprise low basis weight materials and a short cross-directional width causing them to easily fold over themselves and cause issues with application of the absorbent article to a wearer.

US 2013/0211357 discloses taped absorbent articles having a discrete belt portion comprising continuous and discontinuous elastic members arranged in a first zone overlapping the chassis, a second zone overlapping the chassis and including first and second fasteners, and a third zone free of overlap with the chassis.

What is needed are absorbent articles that have reduced ear area fold over to enable easier application by a caregiver.

SUMMARY

The present disclosure solves the ear fold over and application problems of the prior art by providing a taped absorbent article having a rear belt portion with a certain elastic profile, as defined in the claims.

The certain elastic profile allows the ear areas (i.e., portions of the rear belt portion laterally outboard of the leg cuffs) of the rear belt portion to lie in a generally planar configuration when the absorbent article is placed on a flat surface (wearer-facing surface facing away from the flat surface) for much easier application to a wearer. In addition to the certain elastic profile, the rear belt portions are free of overlap with the chassis and contain an integrated waist band region, thereby reducing the tendency for the ear areas to not lie flat during application. Furthermore, the rear belt portion provides a continuous piece of material in the ear areas compared to discrete ears, which provides for reduced ear area fold over owing to longer moment arms and thereby, larger fold over resistance at hinging locations of the ear areas.

The certain elastic profile in the rear belt portion also provides raised regions that correspond approximately with leg cuff areas (from an end view) when the absorbent article is positioned on a flat surface for application. These raised regions can create a bucket-like structure in the absorbent article allowing for easier wearer placement and easier absorbent article application.

The taped absorbent articles of the present disclosure can surprisingly achieve such bucket-like structures with ear panel areas in planar configurations even after compression packaging, which tends to flatten the taped absorbent articles and reduce their three-dimensional structure. Packages of the absorbent articles may have an in-bag stack height in the range of about 70mm to about 100mm, according to the In-Bag Stack Height Test herein, for example.

In a form, the present disclosure is directed, in part, to a taped absorbent article comprising a discrete chassis. The discrete chassis comprises a topsheet, a backsheet, and an absorbent core disposed at least partially intermediate the topsheet and the backsheet. A discrete belt portion is joined to a portion of the discrete chassis. The discrete belt portion comprises a first substrate or nonwoven substrate, a second substrate or nonwoven substrate, and a plurality of elastic members positioned at least partially intermediate the first and second substrates. The elastic members may be elastic strands, strips, or other types of elastic members. The discrete belt portion comprises three zones each comprising at least one of the plurality of elastic members. The three zones extend in a direction generally parallel to a lateral axis of the absorbent article. The three zones comprise a first zone having a first central portion overlapping the chassis, wherein the first zone is positioned most proximal to the lateral axis, a second zone having a second central portion at least partially overlapping the chassis, and a third zone having a third central portion that is at least partially free of, or free of, overlap with the chassis. The first fastener and the second fastener may be at least mostly positioned within the second zone. The third zone is positioned more distal from the lateral axis than the first zone. The second zone is positioned at least partially intermediate the first zone and the third zone. The first zone has a first elastic profile, the second zone has a second elastic profile, and the third zone has a third elastic profile. At least one of the first, second, and third elastic profiles is different than a different one of the first, second, and third elastic profiles.

BRIEF DESCRIPTION OF THE DRAWINGS

The above-mentioned and other features and advantages of the present disclosure, and the manner of attaining them, will become more apparent and the disclosure itself will be better understood by reference to the following description of non-limiting forms of the disclosure taken in conjunction with the accompanying drawings, wherein:

Fig. 1 is a perspective top view photograph of a portion of a prior art taped absorbent article with discrete ears, wearing-facing surface facing the viewer;

Fig. 2 is another perspective top view photograph of a rear portion of the prior art taped absorbent article of Fig. 1, wearing-facing surface facing the viewer;

Fig. 3 is a top perspective view photograph of a rear belt portion of a taped absorbent article of the present disclosure, wearing-facing surface facing the viewer;

Fig. 4 is an end view photograph of the rear belt portion of a taped absorbent article of the present disclosure, wearing-facing surface facing upwards;

Fig. 5 is a plan view of a taped absorbent article of the present disclosure having an elasticized rear belt portion and a front belt portion, wearing-facing surface facing the viewer;

Fig. 6 is a plan view of a rear belt portion and rear portion of a discrete chassis of the taped absorbent article of Fig. 5, wearer-facing surface facing the viewer;

Fig. 7 is a plan view of a taped absorbent article of the present disclosure having an elasticized rear belt portion and an elasticized front belt portion, wearing-facing surface facing the viewer;

Fig. 8 is a plan view of a taped absorbent article having integral fasteners in a rear belt portion of the present disclosure, wearer-facing surface facing the viewer;

Fig. 9 is a plan view of a taped absorbent article of the present disclosure having discrete fasteners that partially extend from a rear belt portion, garment-facing surface facing the viewer;

Fig. 10 is a plan view of a taped absorbent article of the present disclosure having discrete fasteners that extend from a shaped rear belt portion, garment-facing surface facing the viewer;

Fig. 11 is a plan view of a T-shaped taped absorbent article of the present disclosure having discrete fasteners that extend from a rear belt portion, garment-facing surface facing the viewer;

Fig. 12 is a cross-sectional illustration of an absorbent article of the present disclosure taken about line 12-12 of Fig. 5, representing four sections;

Fig. 13 is a simplified view of Fig. 12 representing free body diagrams of the material(s) in each of the four sections, with centroids of the four free body diagrams also illustrated;

Fig. 14A illustrates approximate elastic member contraction forces (using arrows) on the free body diagram of section I of Fig. 13;

Fig. 14B illustrates approximate elastic member contraction forces (using arrows) on the free body diagram of section II of Fig. 13;

Fig. 14C illustrates approximate elastic member contraction forces (using arrows) on the free body diagram of section III of Fig. 13;
Fig. 14D illustrates approximate zero elastic member contraction forces on the free body diagram of section IV of Fig. 13;
Fig. 15 illustrates an example package of a plurality of the taped absorbent articles of the present disclosure;
Figs. 16A-16F are plan views, wearer-facing surfaces facing the viewer, of example rear belt portions and portions of discrete chassises; and
Figs. 17A and 17B are plan views, wearer-facing surfaces facing the viewer, of example rear belt portions and portions of discrete chassises undergoing tension as part of the Determining Regions of Interest Test herein.

DETAILED DESCRIPTION

Various non-limiting forms of the present disclosure will now be described to provide an overall understanding of the principles of the structure, function, manufacture, and use of the taped absorbent article having a belt portion disclosed herein. One or more examples of these non-limiting embodiments are illustrated in the accompanying drawings. Those of ordinary skill in the art will understand that the taped absorbent article having a belt portion described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

As used herein, the term "absorbent article" refers to taped disposable products such as infant, child, or adult diapers, adult incontinence products, and the like which are placed against or in proximity to a body of a wearer to absorb and contain bodily exudates. Typically, these absorbent articles comprise a topsheet, a backsheet, an absorbent core, optionally an acquisition system and/or a distribution system (which may be comprised of one or several layers), and typically other components, with the absorbent core normally placed at least partially between the backsheet and the acquisition and/or distribution system or between the topsheet and the backsheet. For clarity, the term "absorbent articles" does not include pants that are intended to be pulled up a wearer's legs, over the hips and buttocks, and to a wearer's waist.

As used herein, the terms "joined", "bonded", or "attached" encompasses configurations whereby an element is directly secured to another element by affixing the element directly to the other element, and configurations whereby an element is indirectly secured to another element by affixing the element to intermediate member(s) which in turn are affixed to the other element.

As used herein, the term "elastic member" refers to materials exhibiting elastic properties, which include any material that upon application of a force when in its relaxed, initial length may stretch or elongate to an elongated length equal to or greater than 10% more than its initial length and will substantially recover back to about its initial length upon release of the applied force. Elastic members may comprise elastic strands, elastic strips, elastic films, and/or elastic or extensible nonwoven materials or other suitable materials, for example.

The taped absorbent articles having one or more belt portions of the present disclosure will now be described in reference to the various examples set forth in the figures. The taped absorbent articles and rear belt portions thereof may have certain elastic profiles to enable easier application of the absorbent articles to a wearer. The ear areas (i.e., areas laterally outboard of leg cuffs have reduced fold over owing to the certain elastic profiles in the absorbent articles and rear belt portions thereof. One of the features that aid the ear areas of the rear belt portion in having reduced fold over are an integral waist band (i.e., integral to the rear belt portion) that does not overlap with a chassis or leg cuffs and/or leg cuff elastics of the absorbent article. Another feature that provides for reduced ear area fold over is the fact that the rear belt portion in the ear areas creates a longer moment arm compared to discrete ears, thereby requiring a greater force to cause the ear areas to fold over onto themselves. The ear areas may be configured to fold about areas overlapping the leg cuffs because of the three-dimensional bucket-like structure caused by the certain elastic profile of the absorbent articles and the rear belt portions thereof.

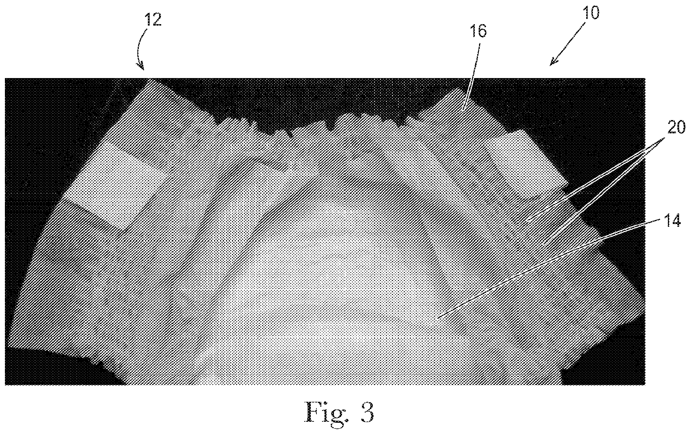

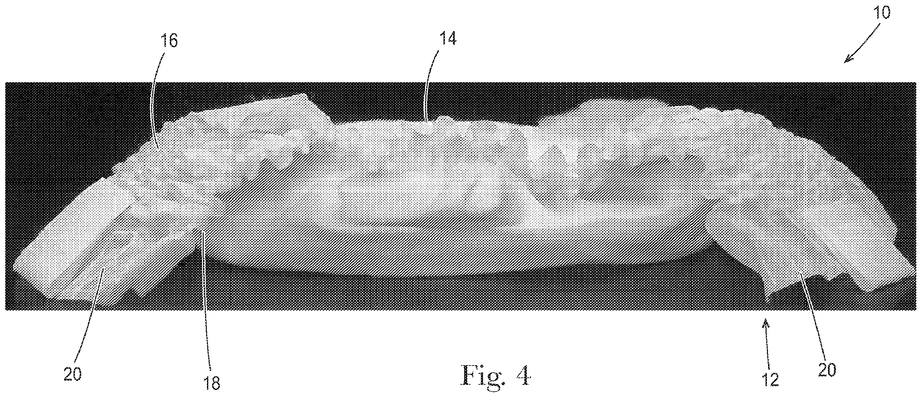

Photographs of a rear belt portion partially overlapped by a discrete chassis of an example absorbent article of the present disclosure are illustrated in Figs. 3 and 4. Fig. 3 is a top perspective view photograph of the rear belt portion overlapped by the discrete chassis of the example absorbent article of the present disclosure. Fig. 4 is an end view of the rear belt portion of Fig. 3. The rear belt portion 12 is joined to a discrete chassis 14. The discrete chassis 14 may be joined to either a wearer or garment facing surface of the rear belt portion 12. The rear belt portion 12 may comprise a first substrate or nonwoven substrate 16, a second substrate or nonwoven substrate 18, and a plurality of elastic members 20 positioned at least partially, or fully, intermediate the first and second nonwoven substrates 16 and 18, respectively. The elastic members 20 may be glued to, bonded to, or otherwise joined to one or both of the first and second substrates 20. Example suitable belt portion constructions are disclosed in U.S. Patent No. 9,326,899, issued on May 3, 2016. In order to facilitate ease of application, without becoming cumbersome, the rear belt portion 12 may have a maximum lateral width of at least 250 mm, at least 300 mm, at least 350 mm, at least 400 mm, at least 450 mm, or at least 500 mm, for example, and a maximum longitudinal length of at least 75 mm, at least 100 mm, at least 125 mm, at least 150 mm, or at least 175 mm, for example. The rear belt portion 12 may comprise integral fasteners (i.e., within the bounds of the rear belt portion 12, although they may be discrete elements attached to the rear belt portion 12) or may comprise fasteners that are joined to the rear belt portion 12 and extend outside of the bounds of the rear belt portion 12. Either of these types of fasteners, or any other suitable types of fasteners, may be used to attach the rear belt portion 12 to a discrete landing zone on a garment-facing surface of a front belt portion of the absorbent article 10, or a garment-facing surface of the front region of the absorbent article 10 depending on the configuration of a particular absorbent article. The fasteners may be at least mostly positioned within a second zone 48 (the second zone 48 is described below), or fully positioned within the second zone 48.

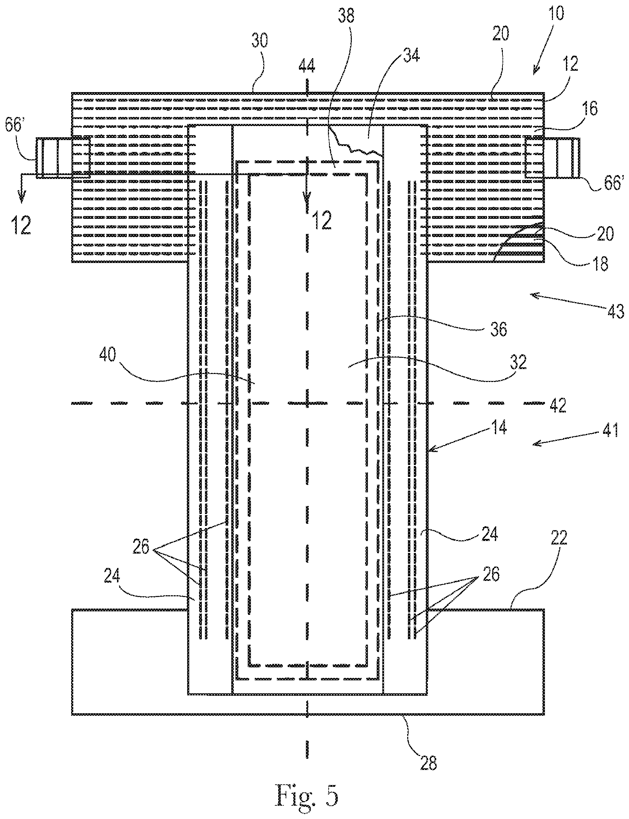

Fig. 5 illustrates a plan view of an example absorbent article 10 of the present disclosure having a discrete chassis 14, a rear belt portion 12, and a front belt portion 22. The absorbent article 10 comprises a lateral axis 42 and a longitudinal axis 44. The rear belt portion 12 may comprise a first substrate or a first nonwoven substrate 16, a second substrate or a second nonwoven substrate 18, and a plurality of elastic members 20. The discrete chassis 14 comprises a front region 41 on a first side of the lateral axis 42 and rear region 43 on a second side of the lateral axis 42. The front belt portion 22 is joined to the front region 41 of the chassis 14 and the rear belt portion 12 is joined of the rear region 43 of the chassis 14. The front belt portion 22 may or may not have elastic members. The rear belt portion 12 has elastic members 20, as are described in greater detail herein. The front belt portion 22 may be free of elastic members or may comprise elastic members. The absorbent article may comprise leg cuffs 24 comprising one or more leg elastics 26. The leg cuffs 24 may have structures detailed in U.S. Patent No. 8,939,957, issued on January 27, 2015. The leg cuffs and/or the leg elastics may not extend to waist edges 28 and 30 of the absorbent article 10. The absorbent article 10 may also comprise a liquid permeable topsheet 32, a liquid impermeable backsheet 34 (that may be breathable), and an absorbent core 36 disposed at least partially intermediate the topsheet 32 and the backsheet 34. The topsheet 32 may be an apertured topsheet, a three-dimensional topsheet that may or may not be apertured, a planar topsheet, or an embossed topsheet, for example. A layer under the topsheet 32, such as an acquisition layer, for example, may have a color (e.g., a blue layer) or an indicia that is visible when viewing the topsheet. The indicia may comprise printed ink or a pigmented adhesive. In some instances, the indicia may be applied to a garment-facing surface of the topsheet 32. The rear belt portion 12 may comprise discrete fasteners 66' as will be described in further detail below.

The absorbent core 36 may comprise an absorbent material. The absorbent material may be enclosed in a core bag 38. The core bag 38 may be formed of two materials that are C-wrapped together, or may have any other suitable configuration. The absorbent material may be free of air felt, or substantially free of air felt (e.g., less than 10%, less than 5% air felt, less than 3%, less than 2%, less than 1%, by weight of the absorbent material, not including the core bag or glues within the core bag), or may comprise a mixture of air felt and superabsorbent polymers. If the core is air felt-free, or substantially air felt-free, the absorbent material or the remainder of the absorbent material may comprise superabsorbent polymers.

The absorbent core 36 may comprise one or more channels. The channels are areas within the core bag that are free of, or substantially free of, the absorbent material. The channels may extend in a generally longitudinal direction in some instances and may or may not be arcuate. The channels may be formed by bonding or gluing one side of the core bag to the other side of the core bag. In other circumstances, embossments may be formed in the absorbent core to give the core the impression of channels. Suitable example absorbent cores are disclosed in U.S. Patent No. 8,979,815, issued on March 17, 2015.

The absorbent article 10 may also comprise an acquisition material 40. The acquisition material 40 may be used to acquire bodily exudates from the topsheet 32 and pass them to the absorbent core 36. The acquisition material 40 may be positioned at least partially intermediate the topsheet 32 and the absorbent core 36. The absorbent article 10 may comprise a distribution layer. The distribution layer may be positioned intermediate the topsheet 32 and the acquisition material 40, between the acquisition layer 40 and the absorbent core 36, or between the absorbent core 36 and the backsheet 34. The distribution layer may comprise pulp or be formed of pulp. In other instances, the distribution material may be a nonwoven material or a material comprising cross-linked cellulosic fibers, for example.

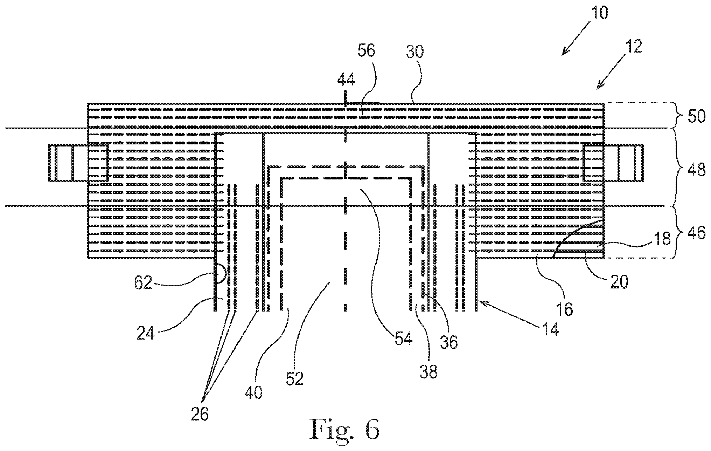

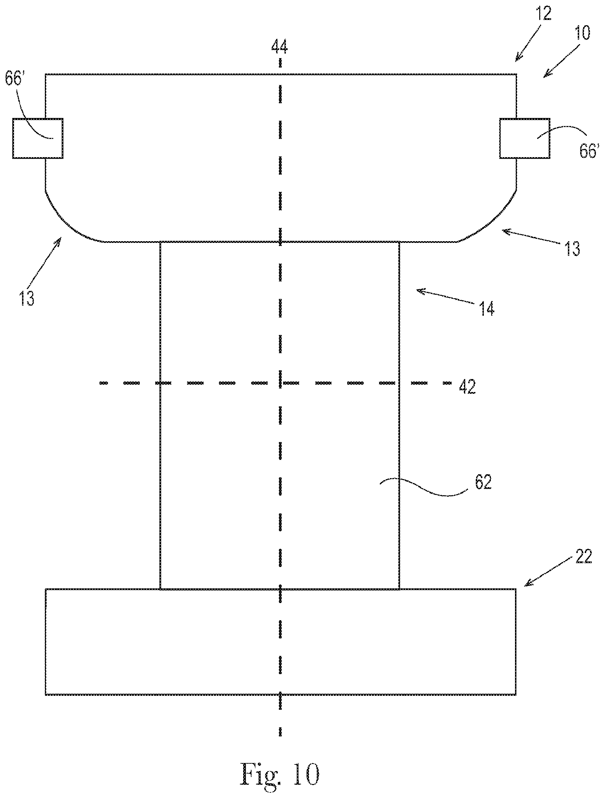

Fig. 6 is a plan view of a portion of the absorbent article 10 of Fig. 5. An example of the overlap of the discrete chassis 14 with the rear belt portion 12 is illustrated. As discussed above, the rear belt portion 12 may comprise the first substrate or the first nonwoven substrate 16, the second substrate or the second nonwoven substrate 18, and the plurality of elastic members 20. The rear belt portion 12 may comprise three zones. Each of the zones may comprise two or more of the plurality of the elastic members 20. At least some of, or all of, the elastic members 20 may extend in a direction generally parallel to, or parallel to, the lateral axis 42. In other instances, the elastic members may be curved elastics or have portions that are curved, but overall even these elastic members will extend generally in in a left to right or right to left direction. The three zones may extend in a direction generally parallel to, or parallel to, the lateral axis 42 of the absorbent article. The three zones may comprise a first zone 46, a second zone 48, and a third zone 50. The three zones on various absorbent articles will be identified as instructed by the Determining Regions of Interest Test herein. These three zones in Fig. 6 are merely to illustrate the concept. Two zones or more than three zones may instead be provided. The first zone 46 may have a first laterally central portion 52 overlapping the discrete chassis 14. The first zone 46 may be positioned most proximal to the lateral axis 42. The second zone 48 may have a second laterally central portion 54 fully, or at least partially, overlapping the chassis 14. In other instances, the second laterally central portion 54 may be free of overlap with the chassis 14. The third zone 50 may have a third central portion 56 that is free of, or at least partially free of, overlap with the chassis 14. The third zone 50 may be the waist band region of the absorbent article 10. The third zone 50 may be positioned most distal from the lateral axis 42. The second zone 48 may be positioned at least partially, or fully, intermediate the first zone 46 and the third zone 50. The first, second, and third zones 46, 48, and 50 of the rear belt portion 12 may have any suitable lateral and longitudinal dimensions. The first zone 46 may be a shaped zone (see e.g., Fig. 10). The second zone 48 may comprise a line of a tension zone owing to the location of the fasteners. The third zone 50 may be a waist band region.

The elastic members 20 may be elastic strands having any suitable cross-sectional shapes, such a rectangular, circular, ovate, and/or semi-circular, for example. In other instances, the elastic members 20 may be strips of an elastic material or film. In still other instances, the elastic members 20 may be elastic films or other stretch or elastic materials. Any combinations of the various elastic members of this paragraph are also within the scope of the present disclosure.

The first zone 46 may have a first elastic profile. The second zone 48 may have a second elastic profile. The third zone 50 may have a third elastic profile. At least two of the first, second, and third elastic profiles are different by at least 10%. In some instances, one of the first, second, and third elastic profiles may be different than a different one of the first, second, and third elastic profiles. For example, the first and second elastic profiles may be the same, while the third elastic profile may be different than the first and second elastic profiles. These different elastic profiles aid in providing the right appearance, ease of application, fit, and/or comfort to the wearer. The elastic profiles may all be different by at least 10%. An elastic profile is based on the elastic member spacing, the elastic member pre-strain, and/or the differences in elastomer basis weights (e.g., decitex).

The first zone 46 may have a first average decitex. The second zone 48 may have a second average decitex. The third zone 50 may have a third average decitex. The first, second, and third average decitexes may be the same or different by at least 10%. In some instances, one of the first average decitex, the second average decitex, and the third average decitex may be different than a different one of the first average decitex, the second average decitex, and the third average decitex by at least 10%. For example, the first average decitex and the second average decitex may be the same, while the third average decitex may be different than the first average decitex and the second average decitex by at least 10%.

Different zones in a belt portion when the absorbent article is positioned on the wearer may perform different functions and may require different elastic profiles. The third zone 50, for example, may be primarily leveraged to provide a seal against the wearer through all of his or her motions, while other zones (e.g., the first and second zones 46 and 48) may provide anchoring support to engage the absorbent article with the wearer. Thus, due to the different functional requirements, and thereby different elastic profiles, of the first, second, and third zones. With the above example, the third zone 50 may have less decitex, pre-strain, and/or a larger elastic spacing compared to the first and second zones 46, 48. Additionally, it will be generally understood by those of skill in the art that a zone may not be homogeneous with regard to decitex as the appearance of transitions between zones may want to be minimized. The average decitex of a zone is determined by the Average Decitex Test herein.

The first zone 46 may have an average first elastic member spacing in a direction parallel to the longitudinal axis 44. The second zone 48 may have a second average elastic member spacing in a direction parallel to the longitudinal axis 44. The third zone 50 may have a third average elastic member spacing in the direction parallel to the longitudinal axis 44. The first, second, and third average elastic member spacing may be the same or different by at least 10%. In some instances, one of the first, second, and third average elastic member spacing may be different than a different one of the first, second, and third average elastic member spacing by at least 10%. For example, the first and second average elastic member spacings may be the same, while the third average elastic member spacing may be different than the first and second average elastic member spacings by at least 10%.

Differences in elastic member spacing in different zones of the rear belt portion 12 may deliver differences in appearance and functional performance. In an instance, it may be desirable to have a zone (e.g., the third zone 50) with a narrower elastic member spacing, thereby allowing more elastic members per area of the zone to increase the resulting forces of the zone.

Example average elastic member spacing may be in the range of about 2mm to about 20mm, about 4mm to about 18mm, about 4mm to about 15mm, about 5mm to about 15mm, about 6mm to about 15mm, greater than 4mm and less than 15mm, about 4mm, about 5mm, about 6mm, about 7mm, about 8mm, about 9mm, or about 10mm, specifically reciting all 0.5mm increments within the specified ranges and all ranges formed therein or thereby. These elastic members may be uniformly spaced or non-uniformly spaced relative to each other in the various zones. In an instance of a front or rear belt portion, the elastics members may be uniformly spaced in portions and non-uniformly spaced in other portions. Average elastic member spacings are measured according to the Average Elastic Member Spacing Test herein.

An elastic member pair is two elastic members that are immediate adjacent to each other and that do not cross a region of interest. Regions of interest are defined in the Determining Regions of Interest Test.

The elastic members 20 in the first zone 46 are discontinuous. The elastic members 20 in the second zone 48 are also discontinuous. The elastic members 20 in the third zone 50 are continuous. The discontinuous elastic members 20 in the first and second zones 46, 48 may partially overlap the chassis 14, but may be free of the first and second central portions 52 and 54 of the chassis 14.

Having discontinuous elastic members 20 in the first zone 46 and at least part of the second zone 48 that are free of the first and second central portions 52 and 54 may be important for a variety of reasons. First, if the elastic members 20 in the first and at least part of the second zones 46 and 48 were continuous and did extend across the first and second central portions 52 and 54, they would cause the rear belt portion 12 and the absorbent article generally to curl towards its longitudinal axis. This curling may cause the absorbent core to also fold toward the longitudinal axis and may cause the leg cuffs to be pulled toward the longitudinal axis. If this elastic member contraction is not pulled out by the caregiver before application of the absorbent article to a wearer, this could potentially lead to increased leakage through the formation of fold lines in the absorbent core and/or leg cuffs not being in a preferred gasketing position. Second, this elastic member overlap of the first and at least part of the first and second central portions 52 and 54 may lead to application issues as an initial width (about the lateral axis 42) of the absorbent article would be reduced, thereby causing the caregiver increased difficulty in finding the fastening tabs that may be hidden underneath the wearer.

At least one of, or all of, the elastic members 20 in the third zone 50 may extend(s) across the longitudinal axis 44 to provide an integral waist band region that at least inhibits, or prevents, the product from gapping at the waist opening during wear.

The elastic members 20 in the first zone 46 may have a first pre-strain. The elastic members 20 in the second zone 48 may have a second pre-strain. The elastic members 20 in the third zone 50 may have a third pre-strain. The first, second, and third pre-strains may be the same or different by at least 10%. In an instance, at least two of the first, second, and third pre-strains may be different by at least 10%. For example, the first and second pre-strains may be the same and may be different than the third pre-strain. The elastic members 20 may be pre-strained in the range of about 25% to about 600%, of about 50% to about 400%, or of about 100% to about 300%, specifically reciting all 0.1% increments within the specified ranges and all ranges formed therein or thereby, for example. Pre-strain is measured using the Pre-Strain Test herein.

In an instance, the rear belt portion 12 may only comprise two zones. A first zone may be a combination of the first zone 46 and the second zone 48 and a second zone may be the third zone 50, for example. In any of the configurations of the taped absorbent articles herein, the first zone 46 may not comprise any elastic members 20. In an instance, the front belt portion 22 may have the same three zones 46, 48, and 50 as the rear belt portion 12. This configuration may be used for better anchoring of the taped absorbent article to a wearer.

Referring again to Fig. 6, the leg cuffs 24 and/or the leg elastics 26 may overlap, or at least partially overlap, some of the plurality of elastic members 20 in the first zone 46 and/or the second zone 48. The leg cuffs 24 and/or the leg elastics 26 may or may not overlap or extend into the third zone 50. The leg cuffs 24 and/or leg elastics 26 may intersect the elastic members 20 at an angle between about 70 degrees and about 110 degrees, between about 80 degrees and about 100 degrees, between about 85 degrees and about 95 degrees, or about 90 degrees, specifically reciting all 0.5 degree increments in the specified ranges and all ranges formed therein or thereby. All angles in this paragraph are with respect to the lateral axis 42 of the absorbent article 10.

The chassis 14 may comprise an outer cover nonwoven material 62 on a garment-facing side of the absorbent article 10. The outer cover nonwoven material 62 may be free of, or at least partially free of, any overlap with the third zone 50. The outer cover nonwoven material 62 may also be free of any overlap with at least a portion of, or all of, the second zone 48 and/or the first zone 46. In such instances, the outer cover nonwoven material 62 may not be coterminous with the backsheet 34. In an instance where a garment-facing surface of the discrete chassis 14 is attached to wearer-facing surfaces of the front and rear belt portions 22 and 12, the outer cover nonwoven material 62 may not be needed to cover the backsheet 34 in the areas of the belt portions 22 and 12, since the backsheet 34 will not be contacting the skin of a wearer. This reduction of the outer cover nonwoven material's length (i.e., not fully overlapping part of the chassis joined to the front and rear belts 22 and 12) can provide cost and material savings to manufacturers and reduced bulk for wearers. In such a form, the outer cover nonwoven material 62 may only slightly overlap with the first zone 46 and a portion of the front belt portion 22 most proximal to the lateral axis 42. This slight overlap of the outer cover nonwoven material 62 and the first zone 46 may reduce unnecessary thickness in the absorbent article without compromising the structural integrity of the absorbent article.

Referring again to Fig. 6, the acquisition material 40, the distribution material (if provided), and/or the absorbent core 36 may at least partially overlap the first zone 46 of the rear belt portion 12 and/or a portion of the front belt portion 22. In other instances, the acquisition material 40, the distribution material, and/or the absorbent core 36 may fully overlap the first zone 46 and may fully or partially overlap the second zone 48. In most instances, the acquisition material 40, the distribution material, and/or the absorbent core 36 may be free of, or at least partially free of, overlap with the third zone 50.

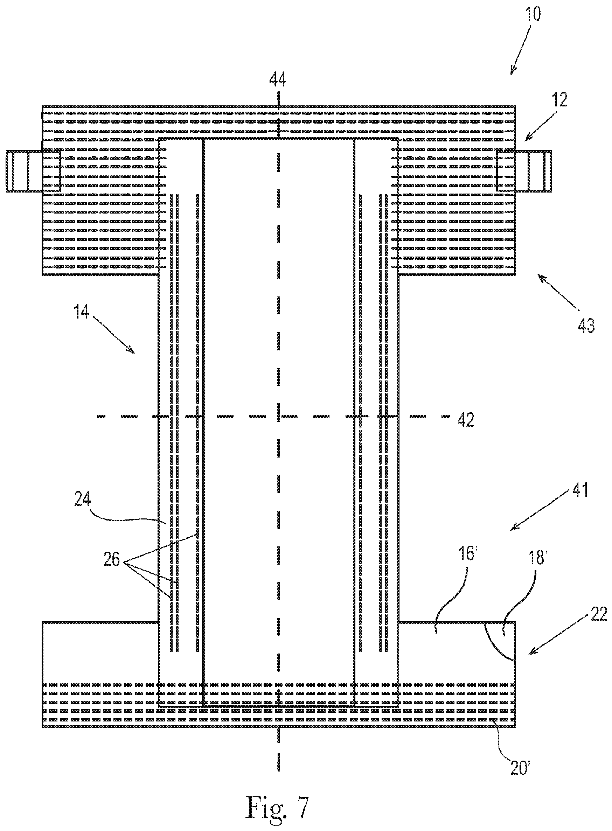

Fig. 7 is a plan view of an absorbent article 10 of the present disclosure, wearer-facing surface facing the viewer. The absorbent article 10 may comprise a front belt portion 22' comprising a plurality of elastic members 20'. The plurality of elastic members 20' may extend in a direction generally along the lateral axis 42, although they may also be curved and/or straight elastics. The elastic members 20' may overlap, not overlap, or partially overlap the front region 41 of the chassis 14. The front belt portion 22' may comprise a first substrate or nonwoven substrate 16', a second substrate or nonwoven substrate 18', and the plurality of elastic members 20' may be positioned at least partially intermediate the first and second substrates 16' and 18'. The leg cuffs 24 and/or the leg elastics 26 may overlap at least some of the elastic members 20' or may be free of overlap with the elastic members 20'.

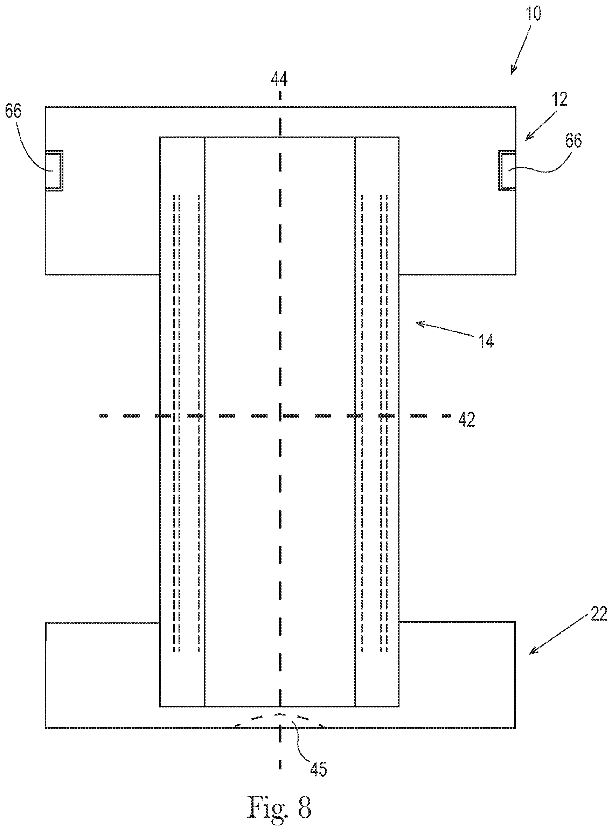

It will be appreciated that the elastic profile of the front belt portion may be different than that of the rear belt portion. The elastic profile of the front belt portion may be different than the elastic profile of the rear belt portion by at least 10%. The same level of contraction and force may not be needed in the front belt portion of the absorbent article as the front belt portion serves a different function than the rear belt portion. The front belt portion may primarily provide application ease, coverage, and/or conformity with a front region of a wearer. The rear belt portion provides these features as well, but also provides at least some of the anchoring properties of the absorbent article. The front belt portion 22 may comprises a belly button notch for infants. The belly button notch may have any suitable shape. An example of such a belly button notch 45 is illustrated in Fig. 8.

Fig. 8 is a plan view of an absorbent article 10 of the present disclosure, wearer-facing surface facing the viewer. The rear belt portion 12 has integral fasteners 66 on a wearer-facing side thereof. The integral fasteners 66 are within the area of the rear belt portion 12, but may be discrete elements attached to the rear belt portion 12. The integral fasteners 66 may be joined to the rear belt portion 12 directly or through a carrier substrate or other material. These integral fasteners 66 are configured to be joined to a landing zone or a landing zone region on a garment facing side of the front belt portion 22. The landing zone region may just be a nonwoven material on a garment-facing side of the front belt portion 22 or may be a discrete landing zone that is attached to the garment-facing surface of the front belt portion 22.

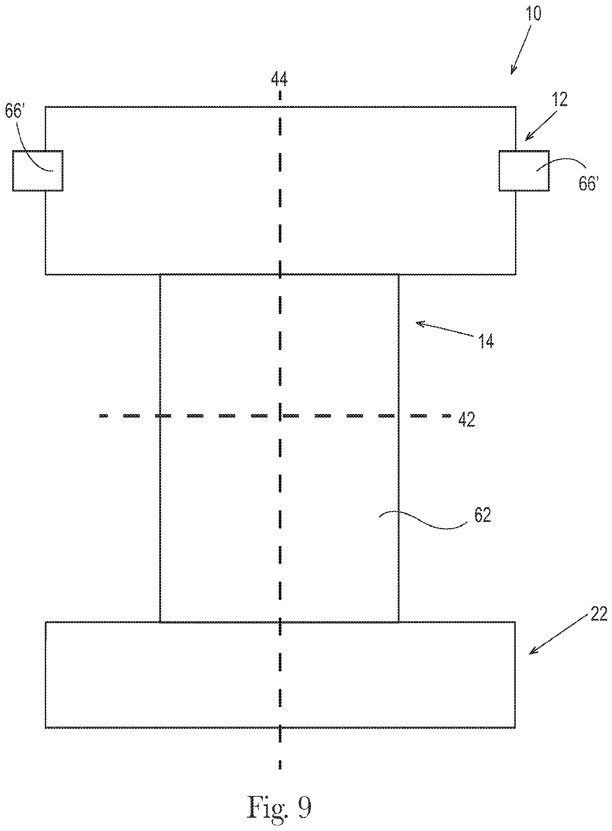

Fig. 9 is a plan view of an absorbent article 10 of the present disclosure, garment-facing surface facing the viewer. The rear belt portion 12 has fasteners 66' that extend beyond the area of the rear belt portion 12. These fasteners 66' are configured to be joined to a landing zone or a landing zone region on a garment-facing side of the front belt portion 22. The fasteners 66' may be joined to either a garment-facing or a wearer-facing surface of the rear belt portion 12 in any suitable location. The fasteners 66' may be joined to the rear belt portion 12 directly or through a carrier substrate or other material.

Fig. 10 is a plan view of an absorbent article 10 of the present disclosure, garment-facing surface facing the viewer. The rear belt portion 12 may have shaped portions 13 or be shaped and still have all of the features discussed herein. The shaping may occur in the first zone 46, the second zone 48, and/or the third zone 50, for example. In other instances, the shaping may only occur in the first zone and possibly in a portion of the second zone 48. See Fig. 6 for the various zones 46, 48, and 50. The front belt portion 22 may also be shaped in various areas. Shaping of the belt portions may remove extra belt portion material to aide in the comfort and fit of the absorbent article.

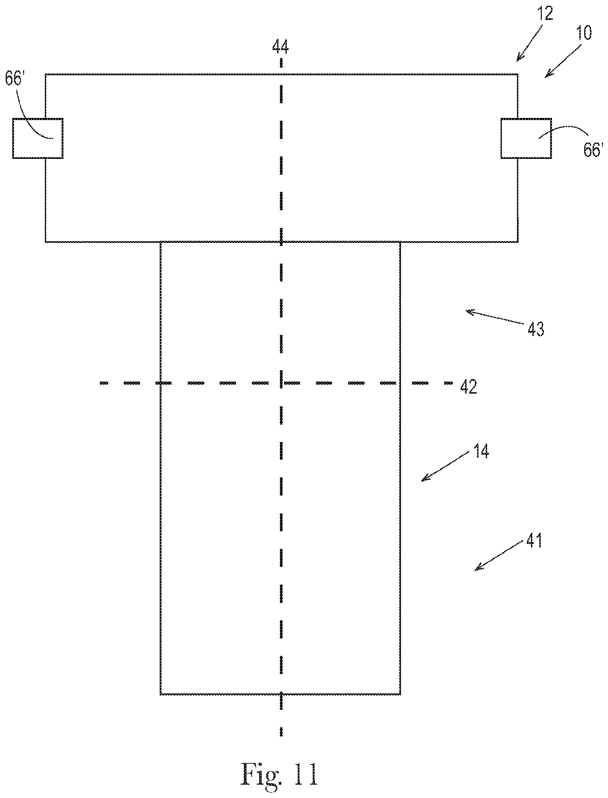

Fig. 11 is a plan view of an absorbent article 10 of the present disclosure, garment-facing surface facing the viewer. The absorbent article 10 may be T-shaped and may not include a front belt portion. In such a configuration, the fasteners 66' would attach to a landing zone or a landing zone region on the garment facing surface of the front portion 41 of the discrete chassis.

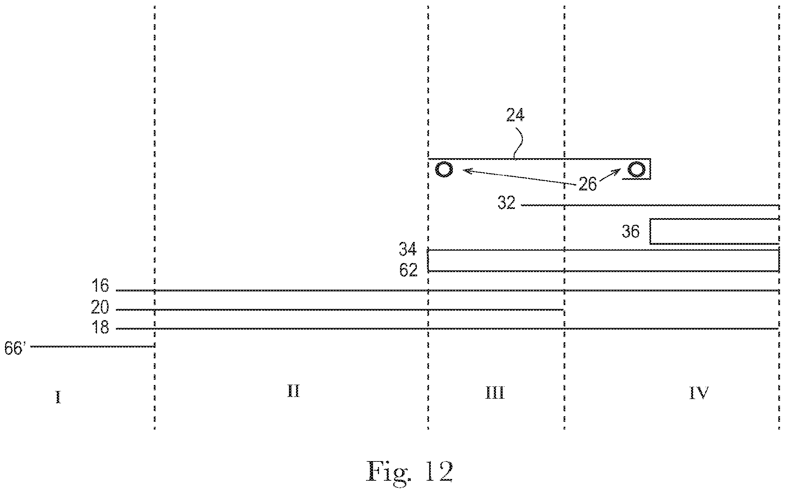

Upon information and belief, Figs. 12-14 describe the technical reasoning behind why the absorbent article 10 of the present disclosure achieves the shape illustrated Figs. 3 and 4, with raised portions proximate to the leg cuffs and in the central region generally and with the ear panel areas remaining generally flat. In a typical absorbent article, such as a diaper, the ear panels tend to want to fold over when lying on a flat surface, wearer-facing surface facing away from the flat surface. This is not desirable as it makes the absorbent article difficult for the caregiver to apply to a wearer lying on his or her back.

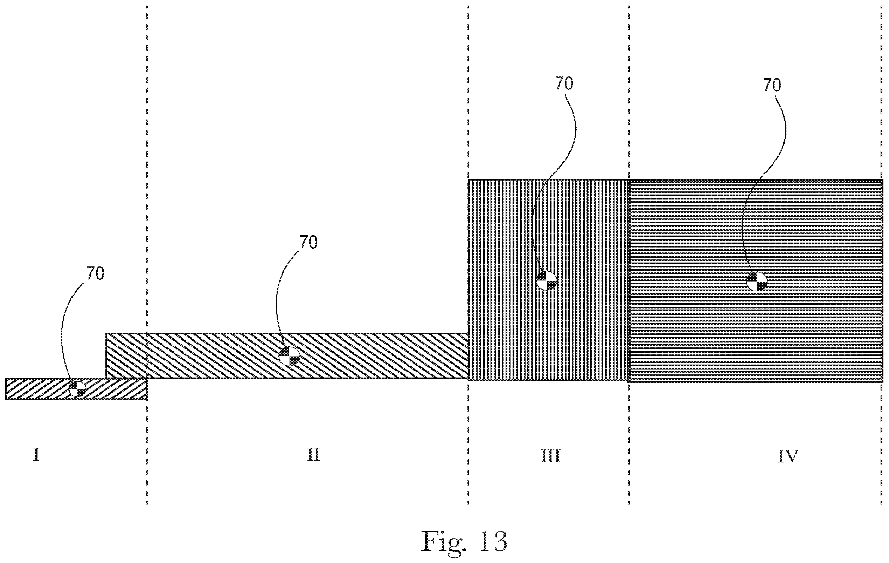

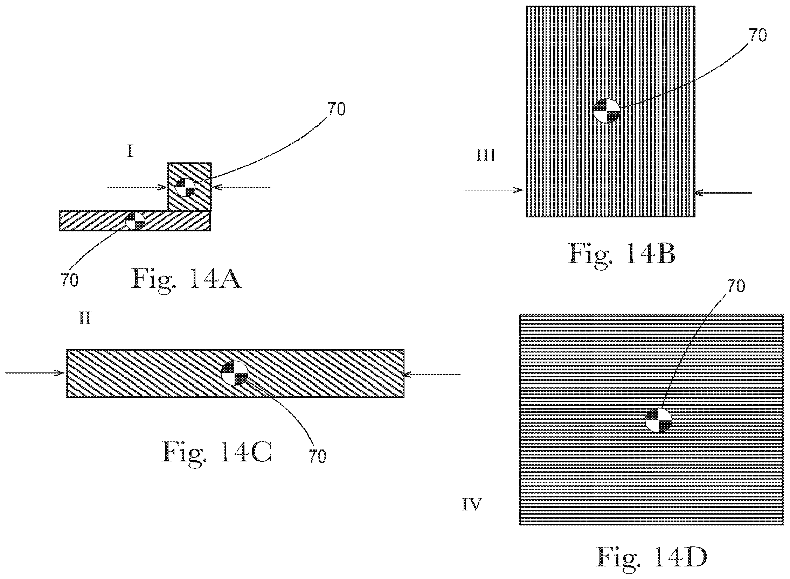

Fig. 12 is a cross-sectional illustration of the absorbent article 10 taken about line 12-12 of Fig. 5, reference numbers remaining the same for the various components. The acquisition layer 40 is not illustrated for purposes of simplicity. The cross-sectional illustration is divided into four distinct sections, labeled I-IV, each one representing a different elastic member contraction profile in the absorbent article 10. The main lateral direction contractive force is provided by the elastic members 20 in section II of the rear belt portion 12. Fig. 13 is a simplified view of Fig. 12 representing free body diagrams of the material in each of the four sections, I-IV. Approximate locations of centroids 70 of the free body diagrams are also illustrated. Note that the centroids of sections III and IV are much higher than the centroids of section II. Fig. 14 illustrates approximate elastic member contraction forces (using arrows) on each free body diagram for each of the four sections, I-IV. It is important to note that for section II, the elastic member contraction forces (shown with arrows) in the rear belt portion 12 are about at the same level of the centroid 70, thereby causing section II of the absorbent article to contract in a generally linear direction toward the centroid 70 and create rugosities that are generally sinusoidal about the neutral axis in the rear belt portion 12. It is also important to note that for section III, the elastic member contraction forces (shown with arrows) of the rear belt portion 12 are much lower than the centroid 70. As a result, section II (i.e., the ear panel area) of the rear belt portion 12 does not have a desire to fold up and over section III and, therefore provides the consumer desired shape illustrated in Figs. 3 and 4. This is quite different than related art absorbent articles where contracting elements such as waistbands are generally placed in sections III and IV above the centroids of the respective sections. This causes the related art absorbent articles to contract at the upper most portions of these sections, which then results in sections I and II contracting up and over section III. Absorbent articles in the related art also sometimes contain attached ears that have limited ability to resist folding over as a result of their shorter width and basis weight compared to the absorbent articles of the present disclosure.

A waist or end edge 30 of the absorbent article 10 may be free of any portion of the chassis 14. When the absorbent article is placed on a flat surface, wearer-facing surface facing the viewer, much like that as illustrated in Fig. 4, the absorbent article may comprise a first portion on a first side of the chassis 14 (i.e., first ear panel area), a second portion on a second side of the chassis 14 (i.e., second ear panel area), and a third central portion in an area along the chassis 14. The third central portion is positioned further away from the flat surface as the first and second portions. The first and second portions also have non-folded over configurations.

Any of the features described with respect to any of the figures (e.g., a front belt portion with elastics) may be combined, as suitable, with other features and/or figures disclosed herein.

## Packages

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Accordingly, packages of the absorbent articles of the present disclosure may have an In-Bag Stack Height of less than about 100 mm, less than about 95 mm, less than about 90 mm, less than about 85 mm, less than about 85 mm, but greater than about 75 mm, less than about 80 mm, less than about 78 mm, less than about 76 mm, or less than about 74 mm, specifically reciting all 0.1 mm increments within the specified ranges and all ranges formed therein or thereby, according to the In-Bag Stack Height Test described herein. Alternatively, packages of the absorbent articles of the present disclosure may have an In-Bag Stack Height of from about 70 mm to about 110 mm, from about 70 mm to about 95 mm, from about 72 mm to about 85 mm, from about 72 mm to about 80 mm, or from about 74 mm to about 78 mm, specifically reciting all 0.1 mm increments within the specified ranges and all ranges formed therein or thereby, according to the In-Back Stack Height Test described herein.

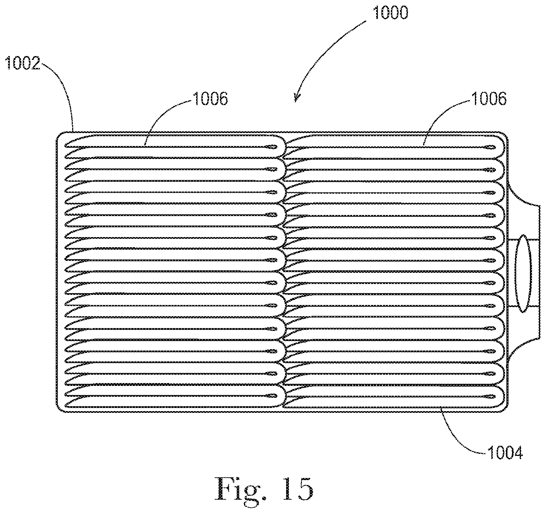

Fig. 15 illustrates an example package 1000 comprising a plurality of absorbent articles 1004. The package 1000 defines an interior space 1002 in which the plurality of absorbent articles 1004 are situated. The plurality of absorbent articles 1004 are arranged in one or more stacks 1006.

### In-Bag Stack Height Test

The in-bag stack height of a package of absorbent articles is determined as follows:

### Equipment

A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within ± 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 ± 1 gram-force (8.34 N) on the absorbent article package, which may be achieved by placing a suitable weight on the center of the non-package-contacting top surface of the horizontal sliding plate so that the total mass of the sliding plate plus added weight is 850 ± 1grams.

### Test Procedure

Absorbent article packages are equilibrated at 23 ± 2 °C and 50 ± 5 % relative humidity prior to measurement.

The horizontal sliding plate is raised and an absorbent article package is placed centrally under the horizontal sliding plate in such a way that the absorbent articles within the package are in a horizontal orientation (see Fig. 15). Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within ± 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) × 10 is calculated and reported to within ± 0.5 mm.

**Common Preconditioning and Measurement Tools:**

Absorbent articles to be tested are preconditioned at 23 °C ± 2 C° and 50% ± 2% relative humidity for 2 hours prior to the testing. All measurements are done using a calibrated ruler accurate to 0.5 mm. All measurements should be recorded to the closest 0.5 mm.

**Determining Regions of Interest Test:**

1. Any leg elastics in the absorbent article are cut or deactivated in order to facilitate the absorbent article laying flat in a planar configuration.

2. The absorbent article is cut in a direction parallel to the lateral central axis along a straight line passing through the most proximal point of the rear belt portion to the lateral axis, across the width of the entire article at that point, using any appropriate cutting means (e.g.: scissors, blades, etc.). This is called the rear belt specimen.

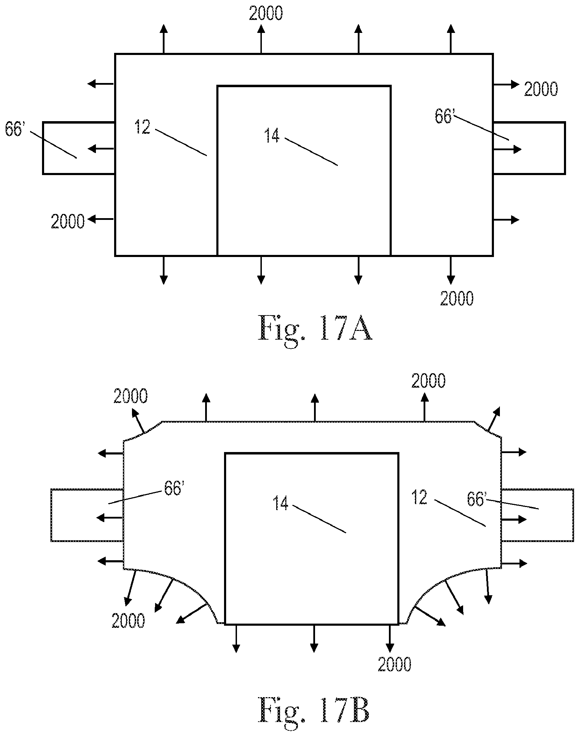

3. The rear belt specimen is attached to a flat, horizontal surface in a flat planar configuration under uniform planar tension with the wearer-facing surface of the diaper facing away from the flat work surface. The rear belt specimen is attached to the work surface by any suitable non-permanent attachment means, such as double-sided tape, while being pulled taut. A force of approximately 1.0 N per centimeter applied perpendicular to the peripheral edge of the rear belt specimen is applied in order to pull the article taut. This may be accomplished by attaching suitable clamps to the peripheral edges of the rear belt specimen and pulling to the desired tension using force gauges or appropriate weight systems. For example, a rectangular rear belt specimen having a longitudinal length of 15.0 cm and a lateral width of 30.0 cm is pulled taut using a force of approximately 15.0 in the longitudinal direction and approximately 30.0 N in the lateral direction, with the forces being uniformly applied along each peripheral edge (see e.g., Fig. 17A.). A non-rectangular shaped rear belt specimen may require multiple clamps and force directions in order to achieve uniform planar tension as shown as an example in Fig. 17B. It is understood when a force is applied to a portion of the peripheral edge the direction of the force may not be purely in the longitudinal or lateral direction. In these instances the applied force will need to be decomposed into its components in the longitudinal and lateral direction to ensure proper tension is being applied. The force is applied gradually over a period of about 10 seconds, and rear belt specimen is secured to the work surface as soon as the desired tension is reached ensuring the dimensions are fixed. It is recognized that the tension in the rear belt specimen may decrease over time due to stress-relaxation effects after the rear belt specimen has been secured to the work surface.

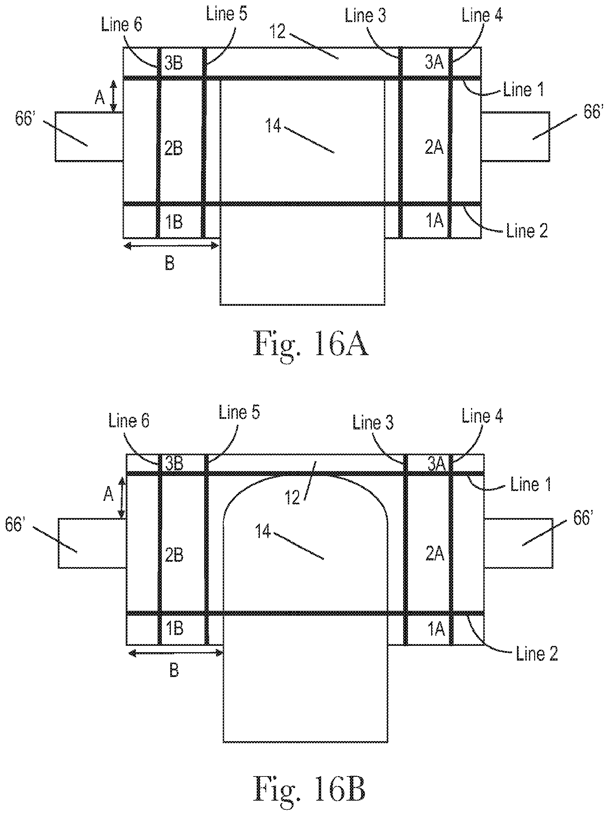

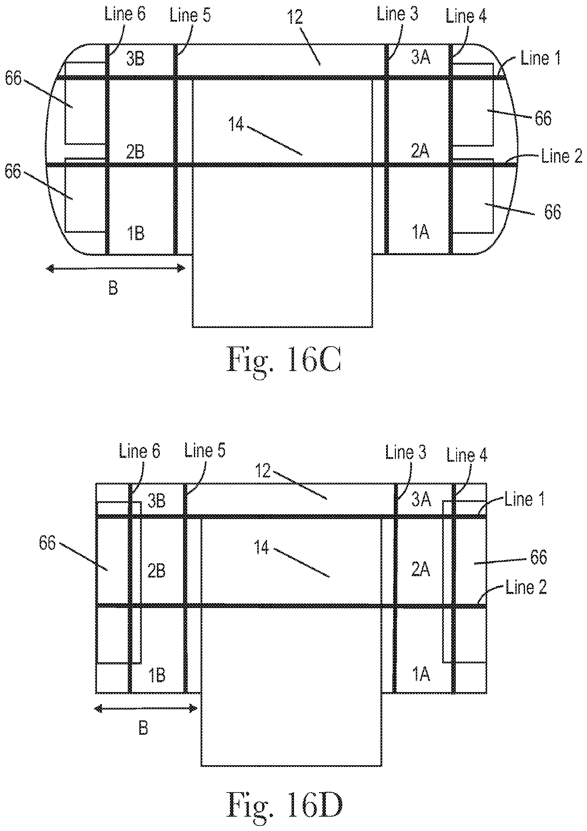

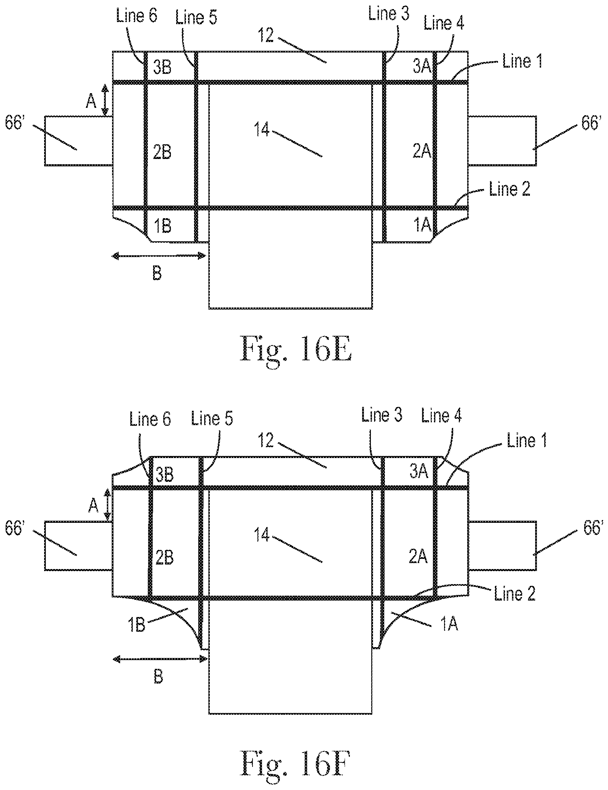

4. On the rear belt specimen, using a marker, draw a first line (line 1 in Figs. 16A-16F) parallel to the central lateral axis of the absorbent article. This first line will run through the most distal point of the discrete chassis relative to the central lateral axis, across the full width of the rear belt specimen. In the event an elastic member pair is not positioned on the side of the first line more distal to the central lateral axis of the absorbent article, the first line shall be moved proximally (i.e., toward the central lateral axis of the absorbent article) in 1 mm increments until one elastic member pair is on the distal side of the second line.

5. Measure the distance, in a direction parallel to the central longitudinal axis of the absorbent article, from the first line to the most longitudinal distal edge of the most longitudinal distal fastener in the rear belt specimen. Record the distance to the nearest 0.5 mm. This distance will be called distance A (see Fig. 16A).

6. On the rear belt specimen, using a marker, draw a second line (line 2 in Fig. 16A-16F) parallel to the central lateral axis of the absorbent article across the entire width of the rear belt specimen. This second line will pass through a point proximal to the central lateral axis, the measured distance A in a direction parallel to the longitudinal axis from the proximal longitudinal edge of the most distal fastener to the lateral central axis. In the event the measured distance is zero or the most longitudinal distal edge of the most longitudinal distal fastener is more distal than line 1, the second line will be drawn parallel to the central lateral axis and pass through a point proximal to the central lateral axis that is 50% of the longitudinal distance between the first line and the most proximal longitudinal edge of the rear belt specimen across the entire width of the rear belt specimen. In the event an elastic member pair is not positioned on the side of the second line more proximal to the central lateral axis of the absorbent article, the second line shall be moved distally (i.e., away from the central lateral axis of the absorbent article) in 1 mm increments until one elastic member pair is on the proximal side of the second line.

7. Measure the lateral distance (i.e., the lateral distance parallel to the lateral axis of the absorbent article) from the most laterally distal edge of the discrete chassis on a first side of the central longitudinal axis of the absorbent article to the most laterally distal edge of the rear belt specimen on the first side of the central longitudinal axis of the absorbent article in a region laterally outboard from the chassis. Record the distance to the nearest 0.5 mm. This distance will be called distance B (see Fig. 16A).

8. Using a marker, draw a third line (line 3 Fig. 16A) distal and parallel to the center longitudinal axis of the absorbent article, on a first side of the central longitudinal axis of the absorbent article, at a lateral distance of 10% of the previously measured distance B of Step 7 outboard from the most laterally distal point the discrete chassis.

9. Using a marker, draw a fourth line (line 4 Fig. 16A) distal and parallel to the center longitudinal axis of the absorbent article, on a first side of the central longitudinal axis of the absorbent article, at a lateral distance of 60% of the previously measured distance B of Step 7 outboard from the most laterally distal point the center chassis.

10. Repeat steps 7-9 for the laterally opposite region outboard of the chassis on a second side of the central longitudinal axis. When the steps are repeated for the laterally opposite region the line drawn in step 8 shall be called the fifth line (line 5 Fig. 16A), the line drawn in step 9 shall be called the sixth line (line 6 Fig. 16A). See Figs. 16A-F for examples with lines 1-6 drawn on them.

- Region of interest 1A will bounded by the second, third and fourth lines, and the most proximal longitudinal edge of the rear belt specimen to the lateral center axis.
- Region of interest 1B will bounded by the second, fifth, and sixth lines, and the most proximal longitudinal edge of the rear belt specimen to the lateral center axis.
- Region of interest 2A will bounded the first, second, third, and fourth lines. In instances where the proximal or distal longitudinal edge or edges of the rear belt specimen are within this region then the region will be bounded by these edges.
- Region of interest 2B will be bounded the first, second, fifth, and sixth lines. In instances where the proximal or distal longitudinal edge or edges of the rear belt specimen are within this region then the region will be bounded by these edges.
- Region of interest 3A will be bounded by the first, third and fourth lines, and the most distal longitudinal edge of the rear belt specimen.
- Region of interest 3B will be bounded by the first, fifth and sixth lines, and the most distal longitudinal edge of the rear belt specimen.

**Average Elastic Member Spacing Test**

In each of the regions 1A, 1B, 2A, 2B, 3A, 3B (from the Determining Regions of Interest Test), measure the longitudinal center to center distance between each elastic member pair at three evenly laterally spaced points within in each region of interest. For example, in a region of interest if there are 5 elastic members present there will be 4 elastic member pairs, provided the elastics do not cross each other within the region of interest. Stated generally, the number of elastic member pairs will be one less than the total number of elastics in the region of interest. Measure this for five products and report the average elastic member spacing for each region of interest to the nearest 0.5 mm.

**Average Decitex Test**

Determine the cross-sectional area orthogonal to the central longitudinal axis of each elastic member by any suitable means (e.g., SEM, optical analysis) within each region of interest defined previously. The elastic member will be present in the laminate and in an un-stretched state. Record the average cross-sectional area to the nearest 0.01 $mm^2$ for each elastic member.

If the density in unknown from the chemical composition of the elastomer it may be determined by conventional means such as a density gradient column, pycnometer or other suitable means. Record the density to the nearest 0.02 g/cc.

Calculate the decitex for each of the elastic members in each of the regions of interest by using the following equation:

$$Decitex \ (g/10000m) = Density \ (g/m^3) \ x \ Cross \ sectional \ Area \ (m^2) \ x \ 10000.$$

Record the average decitex for each region of interest by averaging the decitex of all of the elastic members contained in the region of interest to the nearest 0.1 decitex.

**Pre-Strain Test:**

1. While the rear belt specimen is still attached to the work surface, measure the maximum distance parallel to the central lateral axis, spanned by the longest elastic member in the region of interest. Do this for each of the regions of interest (1A, 1B, 2A, 2B, 3A, 3B-from the Determining Regions of Interest Test) and record to the nearest 0.5 mm. This measurement will be called Measurement C followed by the name of the region of interest. For example this measurement for region of interest 1A will be called C1A, the measurement for region of interest 1B will be called C1B, etc. In instances where the measured elastic member terminates within the region of interest, the distance C may be smaller than the maximum lateral distance of the region of interest. In instances when the elastic member is not parallel to the central lateral axis, the distance C may be less than the end-to-end distance of the elastic member within the region of interest. This should be accomplished within one hour of securing the rear belt specimen to the work surface.

2. Using a suitable cutting instrument, cut a specimen representing each of the regions of interest (i.e., 1A, 1B, 2A, 2B, 3A, 3B) from the product. Specimen 1A will correspond to region of interest 1A, specimen 1B will correspond to region of 1B and so on. These will be referred to as specimens of interest.

3. The specimens of interest are cut and detached from the work surface approximately one hour after securing the rear belt specimen to the work surface.

4. Remove each specimen from being affixed on the work surface and allow it to freely contract on the flat work surface. Allow the specimens to rest for 10 minutes.

5. Repeat the measurement in Step 1 for the same elastic member in each of the specimens. When Step 1 is repeated with the specimen of interest the measurement will be called measurement D followed by the region of interest name. For example, measurement of the same elastic to obtain C1A in the affixed state, will provide measurement D1A in the relaxed state. All measurements should be completed within 1.5 hours from the time the rear belt specimen was secured to the work surface.

6. Calculate the strain using the following equation for all of the measured specimens to the nearest 0.001 decimal. The label for each region of interest has been omitted from the following equation for reading clarity. Strain in region of interest = (Measurement C - Measurement D)/Measurement D. For example if C1A = 60.0 mm, and D1A = 20.0 mm, Strain in region 1A = 2.000.

7. Do this for five total products and independently average the calculated strain for each region of interest.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present disclosure have been illustrated and described, it would be obvious to those skilled in the art that various other changes and modifications may be made without departing from the scope of the present disclosure. It is therefore intended to cover in the appended claims all such changes and modifications that are within the scope of this disclosure.

**Claims**

**1.** A taped absorbent article (10) comprising:

> a lateral axis (42);
> a longitudinal axis (44);
> a front region (41) on a first side of the lateral axis;
> a rear region (43) on a second side of the lateral axis;
> a discrete chassis comprising:
>
> > a topsheet (32);
> > a backsheet (34); and
> > an absorbent core (36) disposed at least partially intermediate the topsheet and the backsheet; and

a discrete belt portion (12) in the rear region and joined to a portion of the discrete chassis, wherein the discrete belt portion comprises:

a first nonwoven substrate (16);
a second nonwoven substrate (18);
a plurality of elastic members (20) positioned at least partially intermediate the first and second nonwoven substrates;
a first fastener (66') on a first side of the longitudinal axis;
a second fastener (66') on a second side of the longitudinal axis; and
three zones each comprising at least one of the plurality of elastic members, wherein the three zones extend in a direction generally parallel to a lateral axis of the absorbent article, wherein the three zones are identified as instructed by the Determining Regions of Interest herein and comprise:

a first zone (46) having a first central portion overlapping the chassis, wherein the first zone is positioned most proximal to the lateral axis;
a second zone (48) having a second central portion at least partially overlapping the chassis, wherein the first fastener and the second fastener are at least mostly positioned within the second zone; and
a third zone (50) having a third central portion that is at least partially free of overlap with the chassis, wherein the third zone is positioned more distal from the lateral axis than the first zone, and wherein the second zone is positioned at least partially intermediate the first zone and the third zone;
wherein the first zone has a first elastic profile;
wherein the second zone has a second elastic profile; and
wherein the third zone has a third elastic profile, wherein at least two of the first, second, and third elastic profiles are different from each other by at least 10%, wherein the elastic profile is based on one selected from elastic member spacing, elastic member pre-strain, and/or the differences in elastomer basis weight, and

wherein the at least one of the plurality of elastic members in the first zone is discontinuous, wherein the at least one of the plurality of elastic members in the second zone is discontinuous, and wherein the at least one of the plurality of elastic members in the third zone is continuous.

2. The taped absorbent article according to Claim 1, wherein the first zone has a first average decitex, wherein the second zone has a second average decitex, wherein the third zone has a third average decitex, and wherein at least two of the first average decitex, the second average decitex, and the third average decitex are different from each other by at least 10%.

3. The taped absorbent article according to Claim 1 or 2, wherein the first zone has a first average elastic member spacing, according to the Average Elastic Member Spacing Test herein, wherein the second zone has a second average elastic member spacing, according to the Average Elastic Member Spacing Test herein, wherein the third zone has a third average elastic member spacing, according to the Average Elastic Member Spacing Test herein, and wherein at least two of the first average elastic member spacing, the second average elastic member spacing, and the third average elastic member spacing are different from each other by at least 10%.

4. The taped absorbent article according to any one of the preceding claims, wherein the at least one of the plurality of elastic members in the first zone has a first pre-strain, wherein the at least one of the plurality of elastic members in the second zone has a second pre-strain, wherein the at least one of the plurality of elastic members in the third zone has a third pre-strain, and wherein at least two of the first, second, and third pre-strains are different from each other by at least 10%.

5. The taped absorbent article according to any one of the preceding claims, wherein the at least one of the plurality of elastic members in the third zone extends across the longitudinal axis, wherein the at least one of the plurality of elastic members in the first zone is free of overlap with the longitudinal axis, and wherein the at least one of the plurality of elastic members in the second zone is free of overlap with the longitudinal axis.

6. The taped absorbent article according to any one of the preceding claims, comprising:
a leg cuff, wherein the leg cuff overlaps a portion of the first zone and a portion of the second zone, and wherein the leg cuff is at least partially free of overlap with the third zone.

7. The taped absorbent article according to Claim 6, wherein the discrete belt portion comprises a waist band portion in the third zone, and wherein the leg cuff does not overlap the waist band portion.

8. The taped absorbent article according to any one of the preceding claims, wherein at least some of the plurality of the elastic members extend in a direction generally parallel to the lateral axis.

9. The taped absorbent article according to any one of Claims 1-7, wherein at least some of the plurality of elastic members are curved elastics.

10. The taped absorbent article according to any one of the preceding claims, wherein the discrete chassis comprises an outer cover nonwoven material, and wherein the outer cover nonwoven material is at least partially free of overlap with the third zone of the discrete belt portion.

11. The taped absorbent article according to any one of Claims 1-9, wherein the discrete chassis comprises an outer cover nonwoven material, and wherein the outer cover nonwoven material is free of overlap with at least the second zone and the third zone of the discrete belt portion.

12. The taped absorbent article according to any one of the preceding claims, wherein the discrete chassis comprises an acquisition material and an absorbent core, and wherein the acquisition material or the absorbent core overlaps at least a portion of the first zone of the discrete belt portion.

13. The taped absorbent article according to any one of the preceding claims, comprising a second discrete belt portion in the front region and joined to a second portion of the discrete chassis, wherein the second discrete belt portion comprises a landing zone or a landing zone region on a garment-facing surface thereof, and wherein the first and second fasteners are configured to engage the landing zone or the landing zone region to form a waist circumference in the taped absorbent article.

14. A package comprising a plurality of the taped absorbent articles according to any one of the preceding claims, wherein the package has an In-Bag Stack Height in the range of about 70 mm to about 100 mm, according to the In-Bag Stack Height Test herein.

**Patentansprüche**

1. Verklebter Absorptionsartikel (10), umfassend:

   eine Querachse (42);
   eine Längsachse (44);
   einen vorderseitigen Bereich (41) auf einer ersten Seite der Querachse;
   einen rückseitigen Bereich (43) auf einer zweiten Seite der Querachse;
   eine separate Grundeinheit, umfassend:

   eine Oberschicht (32);
   eine Unterschicht (34); und
   einen Absorptionskern (36), der wenigstens teilweise zwischen der Oberschicht und der Unterschicht angebracht ist; und

   einen separaten Bundabschnitt (12) in dem rückseitigen Bereich und an einen Abschnitt der separaten Grundeinheit angefügt, wobei der separate Bundabschnitt umfasst:

   ein erstes Vliessubstrat (16);
   ein zweites Vliessubstrat (18);
   eine Vielzahl von Gummibandelementen (20), die wenigstens teilweise zwischen dem ersten und zweiten Vliessubstrat angeordnet sind;
   eine erstes Befestigungsmittel (66') auf einer ersten Seite der Längsachse;
   ein zweites Befestigungsmittel (66') auf einer zweiten Seite der Längsachse; und
   drei Zonen, die jeweils wenigstens eines aus der Vielzahl von Gummibandelementen umfassen, wobei sich die drei Zonen in einer Richtung im Allgemeinen parallel zu einer Querachse des Absorptionsartikels erstrecken, wobei die drei Zonen entsprechend der Anweisung der Prüfung zur Bestimmung der interessierenden Bereiche hierin identifiziert werden und umfassen:

eine erste Zone (46) mit einem ersten Mittelabschnitt, der die Grundeinheit überlappt, wobei die erste Zone am nächsten an der Querachse angeordnet ist;
eine zweite Zone (48) mit einem zweiten Mittelabschnitt, der die Grundeinheit wenigstens teilweise überlappt, wobei das erste Befestigungsmittel und das zweite Befestigungsmittel wenigstens größtenteils innerhalb der zweiten Zone angeordnet sind; und
eine dritte Zone (50) mit einem dritten Mittelabschnitt, der wenigstens teilweise frei von Überlappung mit der Grundeinheit ist, wobei die dritte Zone von der Querachse weiter entfernt angeordnet ist als die erste Zone und wobei die zweite Zone wenigstens teilweise zwischen der ersten Zone und der dritten Zone angeordnet ist;

wobei die erste Zone ein erstes Gummibandprofil aufweist;
wobei die zweite Zone ein zweites Gummibandprofil aufweist; und
wobei die dritte Zone ein drittes Gummibandprofil aufweist, wobei sich wenigstens zwei von dem ersten, zweiten und dritten Gummibandprofil um wenigstens 10 % voneinander unterscheiden, wobei das Gummibandprofil auf einem basiert, ausgewählt aus Gummibandelement-Beabstandung, Gummibandelement-Vorspannung und/oder den Unterschieden in Bezug auf das Elastomerbasisgewicht, und
wobei das wenigstens eine aus der Vielzahl von Gummibandelementen in der ersten Zone unterbrochen ist, wobei das wenigstens eine aus der Vielzahl von Gummibandelementen in der zweiten Zone unterbrochen ist und wobei das wenigstens eine aus der Vielzahl von Gummibandelementen in der dritten Zone ununterbrochen ist.

2. Verklebter Absorptionsartikel nach Anspruch 1, wobei die erste Zone einen ersten durchschnittlichen Decitex aufweist, wobei die zweite Zone einen zweiten durchschnittlichen Decitex aufweist, wobei die dritte Zone einen dritten durchschnittlichen Decitex aufweist und wobei sich wenigstens zwei von dem ersten durchschnittlichen Decitex, dem zweiten durchschnittlichen Decitex und dem dritten durchschnittlichen Decitex um wenigstens 10 % voneinander unterscheiden.

3. Verklebter Absorptionsartikel nach Anspruch 1 oder 2, wobei die erste Zone eine erste durchschnittliche Gummibandelement-Beabstandung gemäß der Prüfung zur durchschnittlichen Gummibandelement-Beabstandung hierin aufweist, wobei die zweite Zone eine zweite durchschnittliche Gummibandelement-Beabstandung gemäß der Prüfung zur durchschnittlichen Gummibandelement-Beabstandung hierin aufweist, wobei die dritte Zone eine dritte durchschnittliche Gummibandelement-Beabstandung gemäß der Prüfung zur durchschnittlichen Gummibandelement-Beabstandung hierin aufweist und wobei sich wenigstens zwei von der ersten durchschnittlichen Gummibandelement-Beabstandung, der zweiten durchschnittlichen Gummibandelement-Beabstandung und der dritten durchschnittlichen Gummibandelement-Beabstandung um wenigstens 10 % voneinander unterscheiden.

4. Verklebter Absorptionsartikel nach einem der vorstehenden Ansprüche, wobei das wenigstens eine aus der Vielzahl von Gummibandelementen in der ersten Zone eine erste Vorspannung aufweist, wobei das wenigstens eine aus der Vielzahl von Gummibandelementen in der zweiten Zone eine zweite Vorspannung aufweist, wobei das wenigstens eine aus der Vielzahl von Gummibandelementen in der dritten Zone eine dritte Vorspannung aufweist, und wobei sich wenigstens zwei von der ersten, zweiten und dritten Vorspannung um wenigstens 10 % voneinander unterscheiden.

5. Verklebter Absorptionsartikel nach einem der vorstehenden Ansprüche, wobei sich das wenigstens eine aus der Vielzahl von Gummibandelementen in der dritten Zone über die Längsachse erstreckt, wobei das wenigstens eine aus der Vielzahl von Gummibandelementen in der ersten Zone frei von Überlappung mit der Längsachse ist und wobei das wenigstens eine aus der Vielzahl von Gummibandelementen in der zweiten Zone frei von Überlappung mit der Längsachse ist.

6. Verklebter Absorptionsartikel nach einem der vorstehenden Ansprüche, umfassend:
ein Beinbündchen, wobei das Beinbündchen einen Abschnitt der ersten Zone und einen Abschnitt der zweiten Zone überlappt und wobei das Beinbündchen wenigstens teilweise frei von Überlappung mit der dritten Zone ist.

7. Verklebter Absorptionsartikel nach Anspruch 6, wobei der separate Bundabschnitt einen Taillenbandabschnitt in der dritten Zone umfasst und wobei das Beinbündchen den Taillenbandabschnitt nicht überlappt.

**8.** Verklebter Absorptionsartikel nach einem der vorstehenden Ansprüche, wobei sich wenigstens einige aus der Vielzahl der Gummibandelemente in einer Richtung im Allgemeinen parallel zu der Querachse erstrecken.

**9.** Verklebter Absorptionsartikel nach einem der Ansprüche 1-7, wobei wenigstens einige aus der Vielzahl von Gummibandelementen gekrümmte Gummibänder sind.

**10.** Verklebter Absorptionsartikel nach einem der vorstehenden Ansprüche, wobei die separate Grundeinheit ein Außenabdeckungsvliesmaterial umfasst und wobei das Außenabdeckungsvliesmaterial wenigstens teilweise frei von Überlappung mit der dritten Zone des separaten Bundabschnitts ist.

**11.** Verklebter Absorptionsartikel nach einem der Ansprüche 1-9, wobei die separate Grundeinheit ein Außenabdeckungsvliesmaterial umfasst und wobei das Außenabdeckungsvliesmaterial frei von Überlappung mit wenigstens der zweiten Zone und der dritten Zone des separaten Bundabschnitts ist.

**12.** Verklebter Absorptionsartikel nach einem der vorstehenden Ansprüche, wobei die separate Grundeinheit ein Aufnahmematerial und einen Absorptionskern umfasst und wobei das Aufnahmematerial oder der Absorptionskern wenigstens einen Abschnitt der ersten Zone des separaten Bundabschnitts überlappt.

**13.** Verklebter Absorptionsartikel nach einem der vorstehenden Ansprüche, umfassend einen zweiten separaten Bundabschnitt in dem vorderseitigen Bereich und an einen zweiten Abschnitt der separaten Grundeinheit angefügt, wobei der zweite separate Bundabschnitt eine Auftreffzone oder einen Auftreffzonenbereich auf einer bekleidungsseitigen Oberfläche davon umfasst, und wobei das erste und das zweite Befestigungsmittel dazu ausgelegt sind, die Auftreffzone oder den Auftreffzonenbereich in Eingriff zu nehmen, um einen Taillenumfang in dem verklebten Absorptionsartikel zu bilden.

**14.** Verpackung, umfassend eine Vielzahl der verklebten Absorptionsartikel nach einem der vorstehenden Ansprüche, wobei die Verpackung gemäß der Stapelhöhe-im-Beutel-Prüfung hierin eine Stapelhöhe im Beutel im Bereich von etwa 70 mm bis etwa 100 mm aufweist.

**Revendications**

**1.** Article absorbant à ruban (10) comprenant :

un axe latéral (42) ;
un axe longitudinal (44) ;
une région antérieure (41) sur un premier côté de l'axe latéral ;
une région arrière (43) sur un deuxième côté de l'axe latéral ;
un châssis discret comprenant :

une feuille de dessus (32) ;
une feuille de fond (34) ; et
une âme absorbante (36) disposée au moins partiellement entre la feuille de dessus et la feuille de fond ; et

une partie de ceinture discrète (12) dans la région arrière et jointe à une partie du châssis discret, dans lequel la partie de ceinture discrète comprend :

un premier substrat non tissé (16) ;
un deuxième substrat non tissé (18) ;
une pluralité d'éléments élastiques (20) positionnés au moins partiellement entre les premier et deuxième substrats non tissés ;
un premier organe de fixation (66') sur un premier côté de l'axe longitudinal ;
un deuxième organe de fixation (66') sur un deuxième côté de l'axe longitudinal ; et
trois zones comprenant chacune au moins l'un de la pluralité d'éléments élastiques, dans lequel les trois zones s'étendent dans une direction généralement parallèle à un axe latéral de l'article absorbant, dans lequel les trois zones sont identifiées tel qu'instruit par le test de détermination de régions d'intérêt ici et comprennent :

une première zone (46) ayant une première partie centrale chevauchant le châssis, dans lequel la première zone est positionnée le plus proximal à l'axe latéral;

une deuxième zone (48) ayant une deuxième partie centrale chevauchant au moins partiellement le châssis, dans lequel les premier et deuxième organes de fixation sont au moins principalement positionnés dans la deuxième zone ; et

une troisième zone (50) ayant une troisième partie centrale qui est au moins partiellement exempte de chevauchement avec le châssis, dans lequel la troisième zone est positionnée plus distale de l'axe latéral que la première zone, et dans lequel la deuxième zone est positionnée au moins partiellement entre la première zone et la troisième zone ;

dans lequel la première zone a un premier profil élastique ;

dans lequel la deuxième zone a un deuxième profil élastique ; et

dans lequel la troisième zone a un troisième profil élastique, dans lequel au moins deux parmi les premier, deuxième, et troisième profils élastiques sont différents l'un de l'autre d'au moins 10 %, dans lequel le profil élastique est basé sur un choisi parmi l'espacement des éléments élastiques, la précontrainte de l'élément élastique, et/ou les différences de masse surfacique élastomère, et

dans lequel l'au moins un de la pluralité d'éléments élastiques dans la première zone est discontinu, dans lequel l'au moins un de la pluralité d'éléments élastiques dans la deuxième zone est discontinu, et dans lequel l'au moins un de la pluralité d'éléments élastiques dans la troisième zone est continu.

2. Article absorbant à ruban selon la revendication 1, dans lequel la première zone a un premier décitex moyen, dans lequel la deuxième zone a un deuxième décitex moyen, dans lequel la troisième zone a un troisième décitex moyen, et dans lequel au moins deux parmi les premier, deuxième et troisième décitex moyens sont différents l'un de l'autre d'au moins 10 %.

3. Article absorbant à ruban selon la revendication 1 ou 2, dans lequel la première zone a un premier espacement moyen des éléments élastiques, selon le test d'espacement moyen des éléments élastiques ici, dans lequel la deuxième zone a un deuxième espacement moyen des éléments élastiques, selon le test d'espacement moyen des éléments élastiques ici, dans lequel la troisième zone a un troisième espacement moyen des éléments élastiques, selon le test d'espacement moyen des éléments élastiques ici, et dans lequel au moins deux parmi les premier, deuxième et troisième espacements moyens des éléments élastiques sont différents l'un de l'autre d'au moins 10 %.

4. Article absorbant à ruban selon l'une quelconque des revendications précédentes, dans lequel l'au moins un de la pluralité d'éléments élastiques dans la première zone a une première précontrainte, dans lequel l'au moins un de la pluralité d'éléments élastiques dans la deuxième zone a une deuxième précontrainte, dans lequel l'au moins un de la pluralité d'éléments élastiques dans la troisième zone a une troisième précontrainte, et dans lequel au moins deux parmi les première, deuxième, et troisième précontraintes sont différentes l'une de l'autre d'au moins 10 %.

5. Article absorbant à ruban selon l'une quelconque des revendications précédentes, dans lequel l'au moins un de la pluralité d'éléments élastiques dans la troisième zone s'étend à travers l'axe longitudinal, dans lequel l'au moins un de la pluralité d'éléments élastiques dans la première zone est exempt de chevauchement avec l'axe longitudinal, et dans lequel l'au moins un de la pluralité d'éléments élastiques dans la deuxième zone est exempt de chevauchement avec l'axe longitudinal.

6. Article absorbant à ruban selon l'une quelconque des revendications précédentes, comprenant :
un revers de jambe, dans lequel le revers de jambe chevauche une partie de la première zone et une partie de la deuxième zone, et dans lequel le revers de jambe est au moins partiellement exempt de chevauchement avec la troisième zone.

7. Article absorbant à ruban selon la revendication 6, dans lequel la partie de ceinture discrète comprend une partie de bande de taille dans la troisième zone, et dans lequel le revers de jambe ne chevauche pas la partie de bande de taille.

8. Article absorbant à ruban selon l'une quelconque des revendications précédentes, dans lequel au moins certains de la pluralité d'éléments élastiques s'étendent dans une direction généralement parallèle à l'axe latéral.

9. Article absorbant à ruban selon l'une quelconque des revendications 1 à 7, dans lequel au moins certains de la pluralité d'éléments élastiques sont des élastiques courbés.

**10.** Article absorbant à ruban selon l'une quelconque des revendications précédentes, dans lequel le châssis discret comprend un matériau non tissé de protection externe, et dans lequel le matériau non tissé de protection externe est au moins partiellement exempt de chevauchement avec la troisième zone de la partie de ceinture discrète.

**11.** Article absorbant à ruban selon l'une quelconque des revendications 1 à 9, dans lequel le châssis discret comprend un matériau non tissé de protection externe, et dans lequel le matériau non tissé de protection externe est exempt de chevauchement avec au moins les deuxième et troisième zones de la partie de ceinture discrète.

**12.** Article absorbant à ruban selon l'une quelconque des revendications précédentes, dans lequel le châssis discret comprend un matériau d'acquisition et une âme absorbante, et dans lequel le matériau d'acquisition ou l'âme absorbante chevauche au moins une partie de la première zone de la partie de ceinture discrète.

**13.** Article absorbant à ruban selon l'une quelconque des revendications précédentes, comprenant une deuxième partie de ceinture discrète dans la région antérieure et jointe à une deuxième partie du châssis discret, dans lequel la deuxième partie de ceinture discrète comprend une zone de réception ou une région de zone de réception sur une surface tournée vers le vêtement de celle-ci, et dans lequel les premier et deuxième organes de fixation sont conçus pour venir en prise avec la zone de réception ou la région de zone de réception pour former un tour de taille dans l'article absorbant à ruban.

**14.** Conditionnement comprenant une pluralité des articles absorbants à ruban selon l'une quelconque des revendications précédentes, dans lequel le conditionnement a une hauteur de pile en sachet dans la plage d'environ 70 mm à environ 100 mm, selon le test de hauteur de pile en sachet ici.

Fig. 1
PRIOR ART

Fig. 2
PRIOR ART

Fig. 3

Fig. 4

Fig. 5

Fig. 6

EP 3 349 706 B1

Fig. 7

Fig. 8

Fig. 9

Fig. 10

Fig. 11

Fig. 12

Fig. 13

I

70

Fig. 14A

70

III

70

Fig. 14B

II

Fig. 14C    70

IV

70

Fig. 14D

EP 3 349 706 B1

Fig. 15

Fig. 16A

Fig. 16B

Fig. 16C

Fig. 16D

35

Fig. 16E

Fig. 16F

Fig. 17A

Fig. 17B

**EP 3 349 706 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130211357 A
- US 9326899 B
- US 8939957 B
- US 8979815 B